(19) Europäisches Patentamt
European Patent Office
Office européen des brevets

(11) Veröffentlichungsnummer: **0 420 092 A1**

# (12) EUROPÄISCHE PATENTANMELDUNG

(21) Anmeldenummer: **90118276.6**

(22) Anmeldetag: **24.09.90**

(51) Int. Cl.5: **C07D 233/56**, A01N 43/50, C07D 233/68

(30) Priorität: **29.09.89 DE 3932552**

(43) Veröffentlichungstag der Anmeldung: **03.04.91 Patentblatt 91/14**

(84) Benannte Vertragsstaaten: **BE CH DE FR GB LI NL**

(71) Anmelder: **BASF Aktiengesellschaft**
**Carl-Bosch-Strasse 38**
**W-6700 Ludwigshafen(DE)**

(72) Erfinder: **Baus, Ulf, Dr.**
**Im Hallgarten 6**
**W-6915 Dossenheim(DE)**
Erfinder: **Reuther, Wolfgang, Dr.**
**Am Pferchelhang 16**
**W-6900 Heidelberg-Ziegelhausen(DE)**

## (54) Verfahren zur Herstellung von 1-Hydroxyimidazol.

(57) Herstellung von 1-Hydroxyimidazolen der allgemeinen Formel I

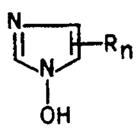

(I)

in der die Reste R gleich oder verschieden sein können und für Halogen oder organische Reste stehen, wobei vicinale reste auch zu einem aromatischen Ring oder einem nicht aromatischen Ring mit 3 bis 12 Ring-C-Atomen verbunden sein können, und n einen Wert von 0 bis 3 hat, indem man ein Imidazol der allgemeinen Formel II

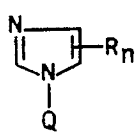

(II)

in der Q für Wasserstoff oder, für den Fall, daß R nicht Halogen bedeutet, ein Alkalimetall- oder ein Erdalkalimetallkation steht, mit einem organischen Peroxid umsetzt.

Die Verfahrensproduckte sind wertvolle Zwischenprodukte für organische Synthesen.

EP 0 420 092 A1

# VERFAHREN ZUR HERSTELLUNG VON 1-HYDROXYIMIDAZOL

Die vorliegende Erfindung betrifft ein neues Verfahren zur Herstellung von 1-Hydroxyimidazolen der allgemeinen Formel I

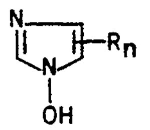

(I)

in der die Reste R gleich oder verschieden sein können und für Halogen oder organische Reste stehen, wobei vicinale Reste auch zu einem aromatischen Ring oder einem nichtaromatischen Ring mit 3 bis 12 Ring-C-Atomen verbunden sein können, und n einen Wert von 0 bis 3 hat.

Aus der DE-A-2 063 857 und der DE-A-2 619 381 sind fungizid und antimykotisch wirksame, in 1-Stellung substituierte Imidazole bekannt.

Man erhält sie, wie auch in Chem. Ber., Band 102 (1969), S. 4177 bis 87, näher beschrieben ist, durch Cyclisierung der entsprechend substituierten Bausteine, z.B. aus Aldehyden, Oximen und Aminen.

Nach diesen Methoden sind jedoch zahlreiche 1-Hydroxyimidazole nicht oder nur schwer zugänglich, so daß der Erfindung ein neues Verfahren zur Herstellung von 1-Hydroxyimidazolen als Aufgabe zugrunde lag, welches es insbesondere gestattet, das bisher nicht bekannte 1-Hydroxyimidazol sowie die Halogenderivate dieser Reihe herzustellen.

Demgemäß wurde ein neues Verfahren zur Herstellung von 1-Hydroxyimidazolen der allgemeinen Formel I

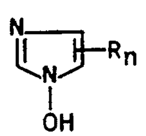

(I)

in der die Reste R gleich oder verschieden sein können und für Halogen oder organische Reste stehen, wobei vicinale Reste zu einem aromatischen Ring oder einem nichtaromatischen Ring mit 3 bis 12 Ring-C-Atomen verbunden sein können, und n einen Wert von 0 bis 3 hat, gefunden, welches dadurch gekennzeichnet ist, daß man Imidazole der allgemeinen Formel II

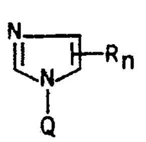

(II)

in der Q vorzugsweise für Wasserstoff oder, insbesondere für den Fall, daß R nicht Halogen bedeutet, ein Alkalimetall- oder ein Erdalkalimetallkation steht, mit einem organischen Peroxid umsetzt.

Generell werden Verbindungen, in denen Q Wasserstoff ist, bevorzugt.

Außerdem wurden die neuen Verbindungen Ia

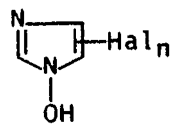

(Ia)

gefunden, in der Hal für gleiche oder verschiedene Halogenatome steht und n einen Wert von 0 bis 3 hat, sowie die Säureadditionssalze von Ia.

Als organische Peroxide eignen sich prinzipiell alle Verbindungen dieser Verbindungsklasse, also z.B. Persäuren wie die Peroxocarbonsäuren Peroxoessigsäure, Peroxobenzoesäure, m-Chlorperoxobenzoesäure, Peroxopropionsäure, Peroxobuttersäure, Peroxomaleinsäure, Monoperoxobernsteinsäure und insbesondere Monoperoxophthalsäure oder Peroxosulfonsäuren wie p-Bromtoluolperoxosulfonsäure, Methylperoxosulfonsäure und insbesondere Toluolperoxosulfonsäure, Peralkohole wie Dipropionylperoxid und Perester wie Diacetylperoxid und insbesondere Dibenzoylperoxid.

Man kann entweder vom freien Imidazol II (Q = H) ausgehen (Variante 1) oder man kann dieses zunächst in das Alkalimetall- oder Erdalkalimetallimidazolid (Q = Alkalimetall oder Erdalkalimetall) überführen und dann weiter mit dem Peroxid umsetzen (Variante 2).

Die Reaktion findet vorzugsweise in stark basischem Milieu statt. Als Basen verwendet man Alkalimetall- und Erdalkalimetallhydroxide, vorzugsweise Natrium- und Kaliumhydroxid. Im folgenden sind diese beiden Ausführungsformen näher erläutert:

Variante 1:

Nach dieser Arbeitsweise können alle Ausgangsverbindungen II eingesetzt werden.

Zur Erzielung eines vollständigen Umsatzes pro Mol des Imidazols II sind mindestens stöchiometrische Mengen des Peroxids erforderlich. Vorzugsweise setzt man Peroxid und Imidazol II jedoch im Molverhältnis von 1,5:1 bis 5:1, besonders 2:1 bis 3:1 um. Es empfiehlt sich, die Umsetzung in einem Lösungs- oder Verdünnungsmittel auszuführen. Hierzu eignen sich alle Flüssigkeiten, die

sich gegenüber Peroxiden im wesentlichen inert verhalten, also z.B. Wasser, Mischungen aus Wasser und Aceton, Tetrahydrofuran, Diethylenglykoldimethylether, Methylenchlorid oder Chloroform. Da die Peroxide sehr reaktiv sind, findet die Reaktion bereits im Temperaturbereich von 0 bis 50 ° C statt.

Höhere Temperaturen, etwa bis zu 80 ° C, bringen im allgemeinen keine Vorteile mehr. Vorzugsweise arbeitet man bei Raumtemperatur.

Variante 2

Diese Ausführungsform ist zu bevorzugen, wenn die eingesetzten Imidazole II sehr teuer sind und man deshalb hohe Ausbeuten von I anstrebt. Allerdings ist sie auf solche Imidazole II beschränkt, die kein Halogen im Molekül enthalten. Aktive Wasserstoffatome, z.B. von Hyroxylgruppen, stören dagegen nicht; vielmehr ist in diesem Fall zur Herstellung der Imidazolide entsprechend mehr der Menge des metallierenden Agens einzusetzen. Metallierende Agentien, mit denen das freie Imidazol II (Q = H) zunächst umgesetzt wird, sind metallorganische Verbindungen, also z.B. Metallalkyle wie n-Butyllithium, tert.-Butyllithium und Methyllithium, Metallaryle wie Phenyllithium, Alkalimetallsuspensionen wie Natrium oder Kalium in Toluol, Alkalimetall-Hydride wie Lithiumhydrid, Kaliumhydrid und insbesondere Natriumhydrid und Erdalkalimetallhydride wie Calciumhydrid.

Zur Erzielung eines vollständigen Umsatzes empfiehlt es sich, ein Verhältnis von Imidazol II zu salzbildendem Reagens von 1:1 bis 10:1 zu wählen. Anschließend setzt man 1 bis 5 mol Peroxid, bezogen auf das Imidazol II, gegebenenfalls unter Bildung des Peroxids in situ aus organischer Verbindung und Wasserstoffperoxid hinzu.

Die Reaktion wird vorzugsweise in einem Lösungsmittel durchgeführt. Hierzu eignen sich alle inerten Lösungsmittel, also z.B. Ether wie Diethylether, Methyl-butylether, Tetrahydrofuran und Dioxan, Glykolether wie Diethylenglykoldimethylether und Triethylenglykoldimethylether, aliphatische Kohlenwasserstoffe wie Pentan, Hexan, Petrolether und Cyclohexan, aromatische Kohlenwasserstoffe wie Benzol, Toluol und Gemische dieser Lösungsmittel.

Aufgrund des Zusatzes eines salzbildenden Stoffes erfolgt die Umsetzung vorzugsweise bei -20 ° C bis +50 ° C, insbesondere bei 0 ° C bis +15 ° C. Das Lösungsmittel wird in einer Menge von 5 bis 50 Gew.-% zugegeben.

Methodisch bietet das erfindungsgemäße Verfahren in beiden Fällen keine Besonderheiten, so daß sich nähere Angaben hierzu erübrigen. Dies gilt auch für Aufarbeitung des Reaktionsgemisches.

Das erfindungsgemäße Verfahren eignet sich zur Herstellung von beliebigen Imidazolen I, wobei die Reste R vorzugsweise folgende Bedeutung haben:

- Wasserstoff;
- Alkyl wie $C_1$-$C_{20}$-Alkyl, bevorzugt $C_1$-$C_8$-Alkyl, besonders bevorzugt $C_1$-$C_4$-Alkyl wie Methyl, Ethyl, n-Propyl, iso-Propyl, n-Butyl, iso-Butyl, sek.-Butyl und tert.-Butyl;
- Halogen wie Fluor, Chlor, Brom und Jod, bevorzugt Chlor, Brom und Jod, besonders bevorzugt Chlor;
- Aryl wie Phenyl, 1-Naphthyl, 2-Naphthyl, 1-Anthranyl, 2-Anthranyl und 9-Anthranyl oder die durch Alkyl und/oder Halogen ein- bis dreifach substituierte Derivate, bevorzugt Phenyl, 1-Naphthyl und 2-Naphthyl, besonders bevorzugt Phenyl.

Die vicinalen Reste können auch zu einem aromatischen Ring oder einem nicht aromatischen Ring mit 3 bis 12 Ring-C-Atomen verbunden sein.

Die 1-Hydroxyimidazole, insbesondere 1-Hydroxyimidazol, sind wertvolle Zwischenprodukte für die Synthese von herbizid und antimykotisch wirksamen imidazolderivaten.

Beispiel 1

Herstellung von 1-Hydroxyimidazol-hydrochlorid

Eine Mischung aus 136 g (2 mol) Imidazol und 2240 g 50 gew.-%iger wäßriger Kaliumhydroxidlösung wurde unter Kühlung mit 435 g (4 mol) Wasserstoffperoxidlösung (30 Gew.-%) versetzt. Danach wurden portionsweise bei <10 ° C 740 g (5 mol) Phthalsäureanhydrid zugesetzt, wonach das Gemisch 24 h gerührt wurde.

Zur Aufarbeitung wurde die Mischung mit Schwefelsäure (pH = 1,0) angesäuert. Der hierbei entstandene Niederschlag wurde abgetrennt, und die zurückbleibende wäßrige Lösung wurde alkalisch gestellt, unter vermindertem Druck eingeengt und anschließend mit dem 0,9- bis 10-fachen Volumen Butanol extrahiert. Die wäßrige Phase wurde wieder angesäuert und mit Butanol extrahiert. Die Butanolphase wurde eingeengt, mit Diethylether versetzt und mit Chlorwasserstoff gesättigt, wobei die Titelverbindung ausfiel und in 2,5 %iger Ausbeute isoliert wurde.

H-NMR (D 20) : 7,19, 7,22 und 8,27 (3s, 3H) [ppm]
C-NMR (D 20) : 118,4, 122,0 und 128,7 (3CH) [ppm]
Smp.: zerfließend

**Ansprüche**

1. Verfahren zur Herstellung von 1-Hydroxyimidazolen der allgemeinen Formel I

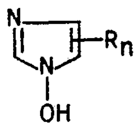

(I)

in der die Reste R gleich oder verschieden sein können und für Halogen oder organische Reste stehen, wobei vicinale Reste auch zu einem aromatischen Ring oder einem nicht aromatischen Ring mit 3 bis 12 Ring-C-Atomen verbunden sein können, und n einen Wert von 0 bis 3 hat, dadurch gekennzeichnet, daß man ein Imidazol der allgemeinen Formel II

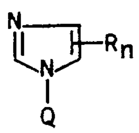

(II)

in der Q für Wasserstoff oder, für den Fall, daß R nicht Halogen bedeutet, ein Alkalimetall- oder ein Erdalkalimetallkation steht, mit einem organischen Peroxid umsetzt.

2. Verfahren nach Anspruch 1, dadurch gekennzeichnet, daß n in den Verbindungen der allgemeinen Formeln I und II den Wert Null hat.

3. 1-Hydroxyimidazole der allgemeinen Formel Ia

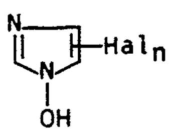

(Ia)

in der Hal für gleiche oder verschiedene Halogenatome steht und n einen Wert von 0 bis 3 hat, sowie die Säureadditionssalze von Ia.

Europäisches Patentamt

# EUROPÄISCHER RECHERCHENBERICHT

Nummer der Anmeldung

EP 90 11 8276

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | HOUBEN-WEYL: "Methoden der Organischen Chemie", Band X/1, 1971, Teil 1, Seiten 1135-1137, Herausgegeben von E. Müller, Georg Thieme Verlag, Stuttgart, DE<br>* Seiten 1135-1137 *<br>– – – | | C 07 D 233/56<br>A 01 N 43/50<br>C 07 D 233/68 |
| P,A | EP-A-0 347 676 (BASF AG)<br>– – – | | |
| P,A | EP-A-0 347 689 (BASF AG)<br>– – – – – | | |
| | | | RECHERCHIERTE SACHGEBIETE (Int. Cl.5) |
| | | | C 07 D 233/00<br>A 01 N 43/00 |

Der vorliegende Recherchenbericht wurde für alle Patentansprüche erstellt

| Recherchenort | Abschlußdatum der Recherche | Prüfer |
|---|---|---|
| Den Haag | 10 Dezember 90 | DE BUYSER I.A.F. |

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze

E: älteres Patentdokument, das jedoch erst am oder nach dem Anmeldedatum veröffentlicht worden ist
D: in der Anmeldung angeführtes Dokument
L: aus anderen Gründen angeführtes Dokument

&: Mitglied der gleichen Patentfamilie, übereinstimmendes Dokument